# EUROPÄISCHE PATENTSCHRIFT

(11) **EP 1 382 338 B1**
(45) Veröffentlichungstag und Bekanntmachung des Hinweises auf die Patenterteilung: **16.07.2008**
(21) Anmeldenummer: 03012869.8
(22) Anmeldetag: 06.06.2003
(51) Int. Cl.: A61K 31/485, A61K 9/22, A61K 47/02, A61K 45/00

(54) **Retardierte Schmerztablette enthaltend Tilidin und ein Lichtschutzmittel**
Controlled release tablet comprising tilidine and a photoprotectant
Comprimé à libération controlée comprenant du tilidine et un photoprotecteur

(30) Priorität: 20.07.2002 DE 10233108
(43) Veröffentlichungstag der Anmeldung: 21.01.2004
(73) Patentinhaber: Krewel Meuselbach GmbH, 53783 Eitorf (DE)
(72) Erfinder: Schierstedt, Dieter, Dr., 53757 St. Augustin (DE)
(74) Vertreter: Jönsson, Hans-Peter

(56) Entgegenhaltungen:
- EP-A- 0 960 619
- DE-A- 19 859 636
- A.H. KIBBE: "Handbook of Pharmaceutical Excipients, Third Edition" 2000 , AMERICAN PHARMACUTICAL ASSOCIATION AND PHARMACEUTICAL PRESS , WASHINGTON DC, LONDON XP002258174 * Seite 565 - Seite 567 *

## Beschreibung

Gegenstand der Erfindung sind feste, peroral applizierbare, Retardiermittel aufweisende Schmerztabletten enthaltend Tilidin und 2-20 Gew.% eines Lichtschutzmittels.

Tilidin, [(+/-)-Ethyl-(trans-2-dimethylamino-phenyl-3-cyclohexen-trans-1-carboxylat)] ist ein entfernt mit Morphin verwandtes Opioidanalgetikum, das enteral rasch resorbiert wird und sich dementsprechend in der Schmerztherapie hervorragend eignet.

In der EP 0 665 830 B1 wird beschrieben, dass vornehmlich Salze des basischen Wirkstoffs für Schmerzmittel in Frage kommen, da die Base als solche keine ausreichende Stabilität über einen längeren Zeitraum aufweist. Es wird weiterhin festgehalten, dass alle Anstrengungen, brauchbare feste Arzneimittelformen des Wirkstoffs Tilidin bereitzustellen, an Stabilitätsproblemen gescheitert sind. Es wird angenommen, dass nur über eine feste Formulierung eine steuerbare Retardierung des Tilidins realisierbar sein dürfte. So wird in der genannten EP 0 665 830 B1 festgehalten, dass Tilidindihydrogenorthophosphat eine hervorragende Stabilität aufweist und sich als bisher einziges Salz für die Herstellung von festen Arzneimittelformen eignet, weil es in fester Form, d. h. in Verbindung mit festen Hilfsstoffen praktisch keine Zersetzung erleidet. Auch soll das Phosphatsalz überraschend pharmazeutisch-technologische Eigenschaften haben, die die Eigenschaften vergleichbarer Tilidinsalze, wie zum Beispiel das Tilidinhydrogensulfat oder das Tilidinhydrogenfumarat übertreffen.

Tilidin wird im Handel üblicherweise zusammen mit einem Morphinantagonisten, beispielsweise Naloxonhydrochlorid eingesetzt.

EP 0 960 619 A1 beschreibt feste peroral applizierbare, Schmerzmittel enthaltend Tilidin als Salz eines organischen nicht toxischen Komplexbildners für 2- oder 3-wertige Metallkationen und einen Morphinantagonisten in einer Cellulosederivatmatrix als Retardiermittel.

DE 198 59 636 A1 beschreibt kontrolliert freisetzende pharmazeutische Zusammensetzungen mit Tilidinmesylat als Wirkstoffe. Im Ausführungsbeispiel 9 wird eine Retardkapsel beschrieben. Diese enthält eine Initialdosis an Tilidinmesylat und eine weitere Retarddosis an Tilidinmesylat, wobei die Retarddosis eine geringe Menge an Titandioxid enthält.

Allen Rezepturen des Standes der Technik ist eine Lichtempfindlichkeit gemeinsam. Es bestand daher die Aufgabe, feste, peroral applizierbare, Retardiermittel aufweisende Schmerztabletten mit verbesserter Lichtbeständigkeit zur Verfügung zu stellen, insbesondere bei denen die in-vitro Freisetzungsrate des Tilidins und des Morphinantagonisten unverändert sind.

Die vorgenannte Aufgabe wurde gelöst durch feste, peroral applizierbare Schmerztabletten, enthaltend ein pharmakologisch unbedenkliches Tilidinsalz, gegebenenfalls eines organischen Komplexbildners für 2- oder 3-wertige Metallkationen, einen Morphinantagonisten und 2-20% Gew.% eines Lichtschutzmittels in einer Cellulosederivatmatrix als Retardiermittel.

Eine weitere Ausführungsform der vorliegenden Erfindung betrifft feste peroral applizierbare Schmerztabletten enthaltend ein pharmakologisch unbedenkliches Tilidinsalz, einen Komplexbildner für 2- oder 3-wertige Metallkationen, einen Morphinantagonisten und ein Lichtschutzmittel in einer Cellulosederivatmatrix als Retardiermittel.

Erfindungsgemäß werden beispielsweise Tilidinsalze von Komplexbildnern 2- oder 3-wertiger Metallkationen eingesetzt. Besonders bevorzugt in diesem Sinne sind organische Säuren, insbesondere Dicarbonsäuren und/oder Tricarbonsäuren, gegebenenfalls hydroxysubstituiert, beispielsweise Citronensäure und/oder Weinsäure bedingt durch die fehlende Toxizität. Wenn die Komplexbildner neben den Tilidinsalzen als weiterer Bestandteil der Schmerztablette enthalten sind, so können diese selbstverständlich auch in Form der Alkali- und/oder Erdalkalimetallsalze enthalten sein. Die genannten Komplexbildner wirken offensichtlich stabilisierend auf das Gesamtsystem und sind in Kombination mit geeigneten Retardiermitteln geeignet, die in-vitro Freisetzungsrate des Tilidins und des Morphinantagonisten im wesentlichen gleichzusetzen.

In gleicher Weise können die Tilidinsalze aber auch in Form der Salze von Mineralsäuren, wie der Chloride, Phosphate oder Sulfate vorliegen. Besonders bevorzugt in diesem Sinne ist das Tilidinhydrochlorid.

Somit ist es im Sinne der vorliegenden Erfindung möglich, Tilidinhydrochlorid-Semihydrat, Tilidinhydrogenfumarat und Tildinhydrogensulfat jeweils mit Naloxonhydrochlorid-Dihydrat, einem Lichtschutzmittel und üblicherweise pharmazeutisch eingesetzten Hilfsstoffen in eine lichtstabile Zubereitung einzubringen. Auch ohne den Zusatz von Komplexbildnern ist in der Cellulosederivat-Matrix eine lichtstabile pharmazeutische Zusammensetzung auf der Basis von Tilidinhydrochlorid-Semihydrat erhältlich. Gleiches gilt selbstverständlich auch für das in der EP 0 665 830 B1 genannte Tilidinhydrogenorthophospat.

Setzt man den vorgenannten Tilidinsalzen Komplexbildner in einer Menge von 0,1 bis 40 Gew.-%, insbesondere 0,2 bis 30 Gew.-%, besonders bevorzugt 2 bis 6 Gew.-%, bezogen auf die Masse der Gesamtzubereitung zu, so ist auch bei Lagerversuchen bei erhöhter Temperatur keine Instabilität und insbesondere kein aggressives Korrosionsverhalten erkennbar.

Der neben Tilidin eingesetzte Morphinantagonist ist im Sinne der vorliegenden Erfindung besonders bevorzugt ausgewählt aus Naloxonhydrochlorid-Semihydrat.

Zur Herstellung retardierter fester Arzneimittelformen des Tilidins kommen prinzipiell alle üblichen Retardierverfahren in Frage, die die Stabilität des Wirkstoffs aufgrund ihrer Zusammensetzung nicht negativ beeinflussen. So sind in der EP 0 043 254 B durch ein Schmelzverfahren retardierte Tabletten beschrieben. Als Retardiermittel eignen sich aber auch prinzipiell schwerlösliche Stoffe, wie zum Beispiel Upide oder lipoide Substanzen, wie Stearinsäure und insbesondere hydriertes Rizinusöl (Cutina®HR) (DE 1 617 657 A, US-4,123,753 A) oder hydrophile Polymere, die als Quellstoffe die Freigabe des Wirkstoffs retardieren (J. Pharm. Sci. S. 974 (1966)). Zur gezielten Steuerung der Freisetzung von Tilidin wird in der EP 0 068 446 B ein Verfahren beschrieben, bei dem die Freigabe der Geschwindigkeit des Wirkstoffs aus einer mittels schwerlöslicher Substanzen retardierten Wirkstoffzubereitung durch die Viskosität eines zugegebenen hydrophilen Polymeren, wie zum Beispiel Methylcellulose oder Carboxymethylcellulose, eingestellt wird, ausgehend von der Erkenntnis, dass die Freigabegeschwindigkeit mit steigender Viskosität zunimmt.

Im Sinne der vorliegenden Erfindung sind daher die Retardiermittel ausgewählt aus hydrophilen oder hydrophoben Polymeren, d. h. Cellulosederivaten, insbesondere Alkylcellulosen oder Hydroxyalkylcellulosen mit 1 bis 6 C-Atomen im Alkyl- oder Hydroxyalkylrest. Besonders bevorzugt im Sinne der vorliegenden Erfindung sind daher die Alkylcellulosen ausgewählt aus Methylcellulose oder Ethylcellulose. In gleicher Weise sind die Hydroxyalkylcellulosen besonders bevorzugt ausgewählt aus Hydroxyethylcellulose oder Hydroxypropylcellulose.

Mit Hilfe der vorliegenden Erfindung konnte ein bisher ungelöstes Problem der Zersetzung durch Lichteinwirkung während des gesamten Herstellungsprozesses der Tabletten (von der Granulierung bis zur Verpressung) gelöst werden.

Überraschenderweise wurde gefunden, dass der Zusatz von 2-20% Gew.% eines Lichtschutzmittels geeignet ist, die Lichtempfindlichkeit der retardierten Schmerztabletten auf der Basis von Tilidin zu verbessern.

Besonders bevorzugt im Sinne der vorliegenden Erfindung werden Lichtschutzmittel eingesetzt, die aus pharmakologisch unbedenklichen Übergangsmetalloxiden der II. und IV. Nebengruppe des Periodensystems der Elemente ausgewählt sind.

Besonders bevorzugt im Sinne der vorliegenden Erfindung enthält das Übergangsmetalloxid Titandioxid oder besteht daraus.

Die Menge der einzusetzenden Lichtschutzmittel richtet sich zum einen nach wirtschaftlichen Gesichtspunkten und zum anderen nach der gewünschten und erforderlichen Lichtschutzwirkung. Eine zu geringe Menge des Lichtschutzmittels hat naturgemäß eine nicht ausreichende Lichtschutzwirkung zur Folge, während eine zu hohe Menge des Lichtschutzmittels aus wirtschaftlichen Erwägungen heraus nicht geeignet ist. Besonders bevorzugt im Sinne der vorliegenden Erfindung enthalten daher die Schmerztabletten 5 bis 10 Gew.-% der Lichtschutzmittel. Diese Lichtschutzmittel werden vorzugsweise während der Mischung der Bestandteile der Tabletten zugegeben, so dass die Tabletten auch während der Granulierung bis zur Pressung lichtunempfindlich sind.

Dadurch wird es gegenüber einer gefilmten Tablette oder einer Kapsel möglich, sowohl während des Mischvorgangs des Verpressens einen optimalen Schutz gegenüber Lichteinwirkung zu gewährleisten.

Wenn im Sinne der vorliegenden Erfindung definiert wird, dass die in-vitro Freisetzungsraten des Tilidins und des Morphinantagonisten im wesentlichen gleich sind, so bedeutet dies, dass die Freisetzungsraten wie folgt bestimmt worden sind:

Die in-vitro Freisetzung von Tilidinsalz und Morphinantagonist aus den Arzneimittelzubereitungen wurde nach DAB10 (Deutsches Arzneibuch, 10. Auflage) in einer Blattrührerapparatur bestimmt. Die Temperatur des Lösungsmediums betrug 37 °C und die Umdrehungsgeschwindigkeit des Rührers 100 U/min. Zu Beginn der Untersuchung wurde jede Tablette in 900 ml künstlichen Magensaft gegeben. Die zu dem vorbestimmten

Zeitpunkt im Lösungsmedium befindliche freigesetzte kombinierte Wirkstoffmenge wurde mittels HPLC bestimmt.

Besonders bevorzugt im Sinne der vorliegenden Erfindung wird die in-vitro Freisetzungsrate demgemäss eingestellt, dass die Freisetzungsgeschwindigkeit des Tilidins nach
1 Stunde 15 bis 30 Gew.-%,
2 Stunden 25 bis 40 Gew.-%,
4 Stunden 50 bis 70 Gew.-%
6 Stunden 60 bis 80 Gew.-% und
8 Stunden 70 bis 90 Gew.-% beträgt.

Darüber hinaus ist es im Sinne der vorliegenden Erfindung besonders bevorzugt, dass sich die Freisetzungsraten von Tilidin und Morphinantagonist nach
1 Stunde um höchstens 25 %, bezogen auf Tilidin,
2 Stunden um höchstens 20 %, bezogen auf Tilidin,
4 Stunden um höchstens 15 %, bezogen auf Tilidin,
6 Stunden um höchstens 12 %, bezogen auf Tilidin und
8 Stunden um höchstens 10 %, bezogen auf Tilidin unterscheiden.

Neben der Retardiermatrix können die erfindungsgemäßen Schmerztabletten auch übliche im Stand der Technik bekannte pharmazeutische Hilfsstoffe enthalten, wie beispielsweise Fette, Fettalkohole, Fettsäuren, Acrylate, Alkylacrylate, Tenside, mikrokristalline Cellulose, Stärken, Milchzucker, Zuckeralkohole, Zucker allgemein oder auch Magnesiumstearat. Das erfindungsgemäße Präparat kann gegebenenfalls auch oberflächenbeschichtet werden.

Die Herstellung der festen, peroral applizierbaren Tabletten, kann nach üblichen im Stand der Technik bekannten Verfahren erfolgen. Hierbei ist beispielsweise die Direktpressung der nicht vorbehandelten Hilfs- und Wirkstoffe zu nennen. Darüber hinaus ist selbstverständlich auch die Granulierung einzelner oder mehrerer Rezepturbestandteile vor der Verpressung möglich.

### Beispiele:

### Ausführungsbeispiel:

Aus 102,9 mg Tilidin x HCI, 8,8 mg Naloxon x HCI, 25 mg Titandioxid, 120 mg Cellulose und Derivate, 10 mg Di-Natriumtartrat-Dihydrat und 1,3 mg Magnesiumstearat wurde durch Direktpressung eine Tablette hergestellt.

### Vergleichsbeispiel:

Analog dem Ausführungsbeispiel unter Weglassen des Titandioxids wurde durch Direktpressung eine Tablette hergestellt.

Die Tabletten des Ausführungsbeispiels und des Vergleichsbeispiels wurden unter folgenden Bedingungen mit Licht bestrahlt.

| | |
|---|---|
| Gerät | "Suntest" |
| Lampenleistung | 765 W/qm |
| Wellenlänge | <800 mm |
| Bestrahlungsdauer | 48 Stunden |

Analytisch konnte nachgewiesen werden, dass die Rezeptur des Vergleichsbeispiels 3,9 mal in Bezug auf Tilidin lichtempfindlicher war als die Rezeptur des Ausführungsbeispiels (Zersetzung Tilidin: 3,1 % zu 0,8 %).

## Patentansprüche

1. Feste, peroral applizierbare Schmerztablette, enthaltend ein pharmakologisch unbedenkliches Tilidinsalz, gegebenenfalls eines organischen Komplexbildners für 2- oder 3-wertige Metallkationen, einen Morphinantagonisten und 2 bis 20 Gew.-% eines Lichtschutzmittels in einer Cellulosederivatmatrix als Retardiermittel.

2. Schmerztablette nach Anspruch 1, **dadurch gekennzeichnet, dass** der Komplexbildner ausgewählt ist aus organischen Säuren, insbesondere Dicarbonsäuren und/oder Tricarbonsäuren, gegebenenfalls hydroxysubstituiert, beispielsweise Citronensäure und/oder Weinsäure.

3. Schmerztablette nach Anspruch 1, enthaltend ein pharmakologisch unbedenkliches Tilidinsalz, einen Komplexbildner für 2- oder 3-wertige Metallkationen, einen Morphinantagonisten und ein Lichtschutzmittel in einer Cellulosederivatmatrix als Retardiermittel.

4. Schmerztablette nach Anspruch 3, **dadurch gekennzeichnet, dass** sich die Säure des Tilidinsalzes von Orthophosphorsäure, Schwefelsäure und/oder Salzsäure ableitet.

5. Schmerztablette nach einem der Ansprüche 1 bis 4, enthaltend Komplexbildner in einer Menge von 0,1 bis 40 Gew.-%, bezogen auf die Masse der Gesamtzubereitung.

6. Schmerztablette nach einem der Ansprüche 1 bis 5, **dadurch gekennzeichnet, dass** der Morphinantagonist Naloxonhydrochlorid ist.

7. Schmerztablette nach einem der Ansprüche 1 bis 6, **dadurch gekennzeichnet, dass** das Retardiermittel ausgewählt ist aus Alkylcellulosen und Hydroxyalkylcellulosen mit 1 bis 6 C-Atomen im Alkyl- oder Hydroxyalkylrest.

8. Schmerztablette nach Anspruch 7, **dadurch gekennzeichnet, dass** die Alkylcellulosen ausgewählt sind aus Methylcellulose und/oder Ethylcellulose.

9. Schmerztablette nach Anspruch 7, **dadurch gekennzeichnet, dass** die Hydroxyalkylcellulosen ausgewählt sind aus Hydroxyethylcellulose und/oder Hydroxypropylcellulose.

10. Schmerztablette nach einem der Ansprüche 1 bis 9, **dadurch gekennzeichnet, dass** das Lichtschutzmittel ausgewählt ist aus pharmakologisch unbedenklichen Übergangsmetalloxiden der II. und IV. Nebengruppe des Periodensystems der Elemente.

11. Schmerztablette nach Anspruch 10, **dadurch gekennzeichnet dass** das Übergangsmetalloxid Titandioxid enthält.

12. Schmerztablette nach Anspruch 10 oder 11, **dadurch gekennzeichnet dass** es 5 bis 10 Gew.-% Lichtschutzmittel, insbesondere Titandioxid enthält.

13. Schmerztablette nach einem der Ansprüche 1 bis 12, **dadurch gekennzeichnet dass** die Freisetzungsgeschwindigkeiten des Tilidins bestimmt nach DAB 10 in einer Blattrührerapparatur bei einer Temperatur des Lösungsmediums von 37°C (900 ml künstlicher Magensaft) und einer Umdrehungsgeschwindigkeit des Rührers von 100 U/min nach
1 Stunde 30 Gew.- %,
2 Stunden 25 bis 40 Gew.-%,
4 Stunden 50 bis 70 Gew.-%,
6 Stunden 60 bis 80 Gew.-% und
8 Stunden 70 bis 90 Gew.-%
betragen.

14. Schmerztablette nach einem der Ansprüche 1 bis 13, **dadurch gekennzeichnet, dass** die in-vitro Freisetzungsrate bestimmt nach DAB 10 in einer Blattrührerapparatur bei einer Temperatur des Lösungsmediums von 37°C (900 ml künstlicher Magensaft) und einer Umdrehungsgeschwindigkeit des Rührers von 100 U/min des Tilidins und des Morphinantagonisten im wesentlichen gleich sind.

15. Schmerztablette nach einem der Ansprüche 1 bis 14, **dadurch gekennzeichnet, dass** sich die Freisetzungsraten von Tilidin- und Morphinantagonist bestimmt nach DAB 10 in einer Blattrührerapparatur bei einer Temperatur des Lösungsmediums von 37°C (900 ml künstlicher Magensaft) und einer Umdrehungsgeschwindigkeit des Rührers von 100 U/min nach
1 Stunde um höchstens 25 %, bezogen auf Tilidin,
2 Stunden um höchstens 20 %, bezogen auf Tilidin,
4 Stunden um höchstens 15 %, bezogen auf Tilidin,
6 Stunden um höchstens 12 %, bezogen auf Tilidin und
8 Stunden um höchstens 10 %, bezogen auf Tilidin
unterscheiden.

## Claims

1. Solid perorally applicable analgesic tablet containing a pharmacologically acceptable tilidine salt, optionally of an organic complexing agent for di- or trivalent metal cations, a morphine antagonist and from 2 to 20% by weight of a light-protecting agent in a cellulose derivative matrix as a retarding means.

2. The analgesic tablet according to claim 1, **characterized in that** said complexing agent is selected from organic acids, especially dicarboxylic acids and/or tricarboxylic acids, optionally hydroxy-substituted, for example, citric acid and/or tartaric acid.

3. The analgesic tablet according to claim 1, containing a pharmacologically acceptable tilidine salt, a complexing agent for di- or trivalent metal cations, a morphine antagonist and a light-protecting agent in a cellulose derivative matrix as a retarding means.

4. The analgesic tablet according to claim 3, **characterized in that** the acid of said tilidine salt is derived from ortho-phosphoric acid, sulfuric acid and/or hydrochloric acid.

5. The analgesic tablet according to any of claims 1 to 4, containing complexing agents in an amount of from 0.1 to 40% by weight, based on the weight of the total formulation.

6. The analgesic tablet according to any of claims 1 to 5, **characterized in that** said morphine antagonist is naloxone hydrochloride.

7. The analgesic tablet according to any of claims 1 to 6, **characterized in that** said retarding means is selected from alkylcelluloses and hydroxyalkylcelluloses having from 1 to 6 carbon atoms in the alkyl or hydroxyalkyl residue.

8. The analgesic tablet according to claim 7, **characterized in that** said alkylcelluloses are selected from methylcellulose and/or ethylcellulose.

9. The analgesic tablet according to claim 7, **characterized in that** said hydroxyalkylcelluloses are selected from hydroxyethylcellulose and/or hydroxypropylcellulose.

10. The analgesic tablet according to any of claims 1 to 9, **characterized in that** said light-protecting agent is selected from pharmacologically acceptable transition metal oxides of the IInd and IVth auxiliary group of the Periodic Table of Elements.

11. The analgesic tablet according to claim 10, **characterized in that** said transition metal oxide contains titanium dioxide.

12. The analgesic tablet according to claim 10 or 11, **characterized by** containing from 5 to 10% by weight of light-protecting agent, especially titanium dioxide.

13. The analgesic tablet according to any of claims 1 to 12, **characterized in that** the release rates of the tilidine as determined according to DAB10 in a blade-agitator apparatus at a temperature of the solvent of 37 °C (900 ml of artificial gastric juice) and a revolutions per minute of the agitator of 100 rpm are:
30% by weight after 1 hour;
from 25 to 40% by weight after 2 hours;
from 50 to 70% by weight after 4 hours;
from 60 to 80% by weight after 6 hours; and
from 70 to 90% by weight after 8 hours.

14. The analgesic tablet according to any of claims 1 to 13, **characterized in that** the in-vitro release rates of tilidine and of the morphine antagonist as determined according to DAB10 in a blade-agitator apparatus at a temperature of the solvent of 37 °C (900 ml of artificial gastric juice) and a revolutions per minute of the agitator of 100 rpm are essentially the same.

15. The analgesic tablet according to any of claims 1 to 14, **characterized in that** the release rates of tilidine and of the morphine antagonist as determined according to DAB10 in a blade-agitator apparatus at a temperature of the solvent of 37 °C (900 ml of artificial gastric juice) and a revolutions per minute of the agitator of 100 rpm are different:
by at most 25%, based on tilidine, after 1 hour;
by at most 20%, based on tilidine, after 2 hours;
by at most 15%, based on tilidine, after 4 hours;
by at most 12%, based on tilidine, after 6 hours; and
by at most 10%, based on tilidine, after 8 hours.

## Revendications

1. Comprimé contre la douleur, solide, pour administration perorale, contenant un sel de tilidine pharmacologiquement inoffensif, éventuellement un agent complexant organique pour des cations métalliques bivalents ou trivalents, un antagoniste de la morphine et 2 à 20 % en poids d'un agent photoprotecteur dans une matrice de dérivé cellulosique en tant qu'agent retard.

2. Comprimé contre la douleur selon la revendication 1, **caractérisé en ce que** l'agent complexant est sélectionné parmi des acides organiques, en particulier des acides dicarboxyliques et/ou des acides tricarboxyliques, éventuellement substitués par un groupe hydroxy, par exemple de l'acide citrique et/ou de l'acide tartrique.

3. Comprimé contre la douleur selon la revendication 1, contenant un sel de tilidine pharmacologiquement inoffensif, un agent complexant pour des cations métalliques bivalents ou trivalents, un antagoniste de la morphine et un agent photoprotecteur dans une matrice de dérivé cellulosique en tant qu'agent retard.

4. Comprimé contre la douleur selon la revendication 3, **caractérisé en ce que** l'acide du sel de tilidine est dérivé de l'acide orthophosphorique, de l'acide sulfurique et/ou de l'acide chlorhydrique.

5. Comprimé contre la douleur selon l'une quelconque des revendications 1 à 4, contenant des agents complexants en une quantité de 0,1 à 40 % en poids, par rapport à la masse de l'ensemble de la préparation.

6. Comprimé contre la douleur selon l'une quelconque des revendications 1 à 5, **caractérisé en ce que** l'antagoniste de la morphine est de l'hydrochlorure de naloxone.

7. Comprimé contre la douleur selon l'une quelconque des revendications 1 à 6, **caractérisé en ce que** l'agent retard est sélectionné parmi des alkylcelluloses et des hydroxyalkylcelluloses ayant 1 à 6 atomes de C dans le radical alkyle ou dans le radical hydroxyalkyle.

8. Comprimé contre la douleur selon la revendication 7, **caractérisé en ce que** les alkylcelluloses sont sélectionnées parmi la méthylcellulose et/ou-l'éthylcellulose.

9. Comprimé contre la douleur selon la revendication 7, **caractérisé en ce que** les hydroxyalkylcelluloses sont sélectionnées parmi l'hydroxyéthylcellulose et/ou l'hydroxypropylcellulose.

10. Comprimé contre la douleur selon l'une quelconque des revendications 1 à 9, **caractérisé en ce que** l'agent photoprotecteur est sélectionné parmi des oxydes métalliques de transition pharmacologiquement inoffensifs des sous-groupes II et IV du système périodique des éléments.

11. Comprimé contre la douleur selon la revendication 10, **caractérisé en ce que** l'oxyde métallique de transition contient du dioxyde de titane.

12. Comprimé contre la douleur selon la revendication 10 ou 11, **caractérisé en ce qu'**il contient 5 à 10 % en poids d'agent photoprotecteur, en particulier du dioxyde de titane.

13. Comprimé contre la douleur selon l'une quelconque des revendications 1 à 12, **caractérisé en ce que** les vitesses de libération de la tilidine, déterminées selon le DAB10 dans un appareil agitateur à pales à une température du milieu de solution (900 ml de sucs gastriques artificiels) de 37°C et à une vitesse de rotation de l'agitateur de 100 tours/min, sont après
1 heure de 30 % en poids,
2 heures de 25 à 30 % en poids,
4 heures de 50 à 70 % en poids,
6 heures de 60 à 80 % en poids, et
8 heures de 70 à 90 % en poids.

14. Comprimé contre la douleur selon l'une quelconque des revendications 1 à 13, **caractérisé en ce que** les taux de libération in vitro de la tilidine et de l'antagoniste de la morphine, déterminés selon le DAB10 dans un appareil agitateur à pales à une température du milieu de solution (900 ml de sucs gastriques artificiels) de 37°C et à une vitesse de rotation de l'agitateur dé 100 tours/min, sont sensiblement identiques.

15. Comprimé contre la douleur selon l'une quelconque des revendications 1 à 14, **caractérisé en ce que** les taux de libération de tilidine et d'antagoniste de la morphine, déterminés selon le DAB10 dans un appareil agitateur à pales à une température du milieu de solution (900 ml de sucs gastriques artificiels) de 37°C et à une vitesse de rotation de l'agitateur de 100 tours/min, se différencient après
1 heure d'au plus 25 %; par rapport à la tilidine,
2 heures d'au plus 20 %, par rapport à la tilidine,
4 heures d'au plus 15 %, par rapport à la tilidine,
6 heures d'au plus 12 %, par rapport à la tilidine et 8 heures d'au plus 10 %, par rapport à la tilidine.
